# EUROPÄISCHE PATENTANMELDUNG

(11) **EP 1 298 136 A2**
(43) Veröffentlichungstag der Anmeldung: **02.04.2003**
(21) Anmeldenummer: 02020595.1
(22) Anmeldetag: 18.09.2002
(51) Int. Cl.: C07F 9/6571, C07F 9/6574, C07B 53/00, C07C 45/50, B01J 31/22

(54) **Chirale Monophosphorverbindungen**

(30) Priorität: 01.10.2001 DE 10148551
(71) Anmelder: BAYER AG, 51368 Leverkusen (DE)
(72) Erfinder: Dreisbach, Claus Dr., 42799 Leichlingen (DE); Meseguer, Benjamin Dr., 50823 Köln (DE); Prinz, Thomas Dr., 51371 Leverkusen (DE); Scholz, Ulrich Dr., 45475 Mühlheim (DE); Militzer, Hans-Christian, 51519 Odenthal (DE); Agel, Friederike, 52068 Aachen (DE); Driessen-Hölscher, Birgit Dr., 52074 Aachen (DE)

(57) **Zusammenfassung**

Die vorliegende Erfindung betrifft Katalysatoren, enthaltend chirale Monophosphorverbindungen, und deren Verwendung, die chiralen Monophosphorverbindungen selbst sowie deren Vorstufen.

## Beschreibung

Die vorliegende Erfindung betrifft Katalysatoren, enthaltend chirale Monophosphorverbindungen, und deren Verwendung, die chiralen Monophosphorverbindungen selbst sowie deren Vorstufen. Chirale Monophosphorverbindungen sind im Rahmen der Erfindung insbesondere chirale Monophosphite, Monophosphoramidite und Monophosphonite.

Es ist bereits bekannt, chirale Monophosphite bzw. deren Übergangsmetall-Komplexe für asymmetrische Synthesen einzusetzen (vgl. auch A. Alexakis, Tetrahedron Asymmetry, 1997, 8, 3193-3196; C. Claver et al., Chem. Commun., 2000, 2383-2384; W. Chen, J. Xiao, Tetrahedron Letters, 42, 2001, 2897 bis 2899; M. Reetz, G. Mehler, Angew. Chem., 2000, 112, 4047 bis 4049).

Der Einsatz von chiralen Monophosphoramiditen bzw. deren Übergangsmetall-Komplexen in asymmetrischen Synthesen ist beispielsweise aus van den Berg et al., J. Am. Chem. Soc., 2000, 122, 11539 bis 11540 und H. Waldmann, Chem. Eur. J. 2000, 6, 671-675, der Einsatz von chiralen Monophosphoniten aus C. Claver et al., Chem. Commun., 2000, 961 bis 962 bekannt.

Allen bislang bekannten chiralen Liganden ist jedoch gemeinsam, dass sie sich vom Grundgerüst des 2,2'-Dihydroxy-1,1'-binaphthyl oder anderer mehrkerniger Dihydroxy-Bisaryle ableiten. Der Nachteil solcher Liganden ist, dass zur Variation der elektronischen und sterischen Eigenschaften nur begrenzte Substitutionsmöglichkeiten zur Verfügung stehen. Der Einsatz für verschiedene asymmetrische Reaktionstypen und die Anwendbarkeit auf eine Vielzahl von Substraten macht jedoch breite Substitutionsmöglichkeiten wünschenswert.

Weiterhin bestand das Bedürfnis, Katalysatoren zu entwickeln, die insbesondere in der Anwendung in asymmetrischen Hydrogenierungen neben hoher Enantioselektivität auch hohe Umsatzraten bei milden bis moderaten Reaktionsbedingungen ermöglichen.

Überraschenderweise wurde nun gefunden, dass chirale Monophosphorverbindungen der allgemeinen Formel (I) bzw. auf diesen basierende Katalysatoren für asymmetrische Synthesen besonders geeignet sind, 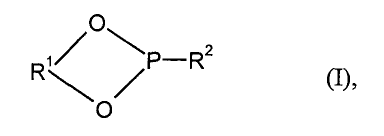 in der
- R¹: für einen unsubstituierten oder substituierten 1,1'-Biphenyl-2,2'-diyl-Rest steht und R² für einen Rest steht, der ausgewählt ist aus der Gruppe substituiertes oder unsubstituiertes Alkyl, substituiertes oder unsubstituiertes Aryl, substituiertes oder unsubstituiertes Alkoxy, substituiertes oder unsubstituiertes Aryloxy oder tertiäres Amino.

Bevorzugt steht R² für substituiertes oder unsubstituiertes Alkoxy oder substituiertes oder unsubstituiertes Aryloxy oder tertiäres Amino, besonders bevorzugt für substituiertes oder unsubstituiertes Alkoxy oder substituiertes oder unsubstituiertes Aryloxy.

Unsubstituiertes oder substituiertes Alkyl steht beispielsweise und bevorzugt für unverzweigte, verzweigte, cyclische oder acyclische C₁-C₁₈-Alkylreste, die entweder nicht oder zumindest teilweise durch Fluor, Chlor, Brom, Oxo, Hydroxy, unsubstituiertes oder substituiertes Aryl, C₁-C₆-Alkoxy, wie zum Beispiel Methoxy, Ethoxy, iso-Propoxy oder n-Propoxy, n-Butoxy, oder tert.-Butoxy, primäres, sekundäres oder tertiäres Amino, Cyano oder Carboxylgruppen oder Derivaten davon substituiert sind. Als Beispiele für Derivate von Carboxylgruppen seien Ester, Amide oder Salze genannt.

Besonders bevorzugt steht unsubstituiertes oder substituiertes Alkyl für verzweigte, cyclische oder acyclische C₃-C₁₂-Alkylreste, die entweder nicht oder zumindest teilweise durch Fluor, Oxo, Hydroxy, Methoxy, Ethoxy, Phenyl, 2-Methoxyphenyl, 2-Pyridyl, 3-Pyridyl, 4-Pyridyl, Amino, Dimethylamino, Diethylamino, Diisopropylamino, Cyano- oder Carboxylgruppen, deren Natrium- oder Kaliumsalz oder deren Methyl- oder Ethyl-Ester oder deren Amid, Dimethylamid oder Diethylamid substituiert sind.

Ganz besonders bevorzugt steht unsubstituiertes oder substituiertes Alkyl für IsoPropyl, tert.-Butyl, Cyclohexyl, 1-Butyl, 2-Butyl, 2-Ethylhex-1-yl, Benzyl, 2-Methoxybenzyl, 2-Pyridylmethyl, 1-Phenylethyl.

Unsubstituiertes oder substituiertes Alkoxy steht beispielsweise und bevorzugt für unverzweigte, verzweigte, cyclische oder acyclische C₁-C₁₈-Alkoxyreste, die entweder nicht oder zumindest teilweise durch Fluor, Chlor, Brom, Oxo, freies oder geschütztes Hydroxy, C₁-C₆-Alkoxy, wie zum Beispiel Methoxy, Ethoxy, iso-Propoxy oder n-Propoxy, n-Butoxy oder tert.-Butoxy, substituiertes oder unsubstituiertes C₆-C₁₀-Aryl, wie zum Beispiel Phenyl oder 2-Pyridyl, primäres, sekundäres oder tertiäres Amino, Cyano oder Carboxylgruppen oder Derivaten davon substituiert sind.

Besonders bevorzugt steht unsubstituiertes oder substituiertes Alkoxy für unverzweigte, verzweigte, cyclische oder acyclische C₂-C₁₂-Alkoxyreste, die entweder nicht oder zumindest teilweise durch Fluor, Chlor, freies oder geschütztes Hydroxy, substituiertes oder unsubstituiertes Phenyl, 2-Pyridyl, C₁-C₆-Alkoxy, wie zum Beispiel Methoxy, Ethoxy, iso-Propoxy oder n-Propoxy, n-Butoxy oder tert.-Butoxy, C₁-C₆-Dialkylamino, C₁-C₆-Alkylcarbonylamino, Benzoylamino, 4-Methylphenylsulfonylamino, Imidazoyl, Phthalimidyl, C₁-C₆-Alkyloxycarbonyl, C₁-C₄-Dialkylaminocarbonyl substituiert sind.

Ganz besonders bevorzugt steht unsubstituiertes oder substituiertes Alkoxy für unverzweigte, verzweigte, cyclische oder acyclische C₂-C₆-Alkoxyreste, die entweder nicht oder zumindest teilweise durch Fluor, Chlor, freies oder geschütztes Hydroxy, einfach oder zweifach substituiertes oder unsubstituiertes Phenyl, 2-Pyridinyl, C₁-C₄-Dialkylamino, C₁-C₄-Alkylcarbonylamino, Benzoylamino, 4-Methylphenyl-sulfonylamino, Imidazoyl, Phthalimidyl, C₁-C₄-Alkyloxycarbonyl, C₁-C₄-Dialkylaminocarbonyl, C₁-C₄-Alkoxy, wie zum Beispiel Methoxy, Ethoxy, iso-Propoxy oder n-Propoxy, n-Butoxy oder tert.-Butoxy, substituiert sind.

Noch weiter bevorzugt steht unsubstituiertes oder substituiertes Alkoxy für Methoxy, Ethoxy, iso-Propoxy, Cyclohexyloxy, Phenoxy, (R)-1-Phenylethoxy oder (S)-1-Phenylethoxy.

Substituiertes oder unsubstituiertes Aryl steht beispielsweise und bevorzugt für carbocyclische aromatische Reste mit 6 bis 18 Gerüstkohlenstoffatomen oder heteroaromatische Reste mit 5 bis 18 Gerüstkohlenstoffatomen, in denen keines, ein, zwei oder drei Gerüstkohlenstoffatome pro Cyclus, im gesamten Molekül mindestens jedoch ein Gerüstkohlenstoffatom, durch Heteroatome, ausgewählt aus der Gruppe Stickstoff, Schwefel oder Sauerstoff, substituiert sein können. Weiterhin können die carbocyclischen aromatischen Reste oder heteroaromatische Reste mit bis zu fünf gleichen oder verschiedenen Substituenten pro Cyclus substituiert sein, ausgewählt aus der Gruppe freies oder geschütztes Hydroxy, Iod, Brom, Chlor, Fluor, Cyano, freies oder geschütztes Formyl, C₁-C₁₂-Alkyl wie zum Beispiel Methyl, Ethyl, n-Propyl, iso-Propyl, n-Butyl, tert.-Butyl, Cyclohexyl, n-Hexyl, n-Octyl oder iso-Octyl, C₆-C₁₂-Aryl, wie zum Beispiel Phenyl, Tri(C₁-C₆-alkyl)siloxyl wie zum Beispiel Trimethylsiloxyl, Triethylsiloxyl oder Tri-n-butylsiloxyl oder Resten der allgemeinen Formel (II),

A-B-D-E (II),

in der unabhängig voneinander
- A: fehlt oder für einen C₁-C₈-Alkylenrest wie zum Beispiel Methylen, 1,2-Ethylen, 1,1-Ethylen, 1,3-Propylen, 1,2-Propylen, 1,4-Butylen oder 2,3-Butylen steht und
- B: fehlt oder für Sauerstoff, Schwefel oder NR³ steht,
wobei
R³ Wasserstoff, C₁-C₁₆-Alkyl wie beispielsweise Methyl, Ethyl, n-Propyl, iso-Propyl, n-Butyl, tert.-Butyl, Cyclohexyl, n-Hexyl, n-Octyl oder iso-Octyl oder C₆-C₁₀-Aryl wie beispielsweise Phenyl oder 2-, 3- oder 4-Tolyl bedeutet und
D für eine Carbonyl-Gruppe steht und
E für R⁴, OR⁴, NHR⁵ oder N(R⁵R⁶)₂ steht,
wobei
R⁴ für C₁-C₁₂-Alkyl, C₆-C₁₀-Aryl und
R⁵ und R⁶ jeweils unabhängig für C₁-C₈-Alkyl, oder C₆-C₁₀-Aryl oder N(R⁵R⁶)₂ zusammen für einen cyclischen Aminorest stehen,
oder Resten der allgemeinen Formeln (IIIa) und (IIIb)

A-E (IIIa)

A-COX (IIIb)

in denen A und E die oben angegebene Bedeutung besitzen und X für OH, NH₂ oder OM steht, wobei M ein Alkalimetallion, ein halbes Äquivalent eines Erdalkalimetallions, ein Ammoniumion oder ein organisches Ammoniumion bedeuten kann.

Beispiele für carbocyclische aromatische Reste mit 6 bis 18 Gerüstkohlenstoffatomen sind zum Beispiel Phenyl, Naphtyl, Phenanthrenyl, Anthracenyl oder Fluorenyl, heteroaromatische Reste mit 5 bis 18 Gerüstkohlenstoffatomen, in denen keines, ein, zwei oder drei Gerüstkohlenstoffatome pro Cyclus, im gesamten Molekül mindestens jedoch ein Gerüstkohlenstoffatom, durch Heteroatome, ausgewählt aus der Gruppe Stickstoff, Schwefel oder Sauerstoff, substituiert sein können, sind beispielsweise Pyridinyl, Oxazolyl, Thiophen-yl, Benzofuranyl, Benzothiophen-yl, Dibenzofuranyl, Dibenzothiophen-yl, Furanyl, Indolyl, Pyridazinyl, Pyrazinyl, Pyrimidinyl, Triazolyl oder Chinolinyl.

Geschütztes Formyl steht im Rahmen der Erfindung für einen Formyl-Rest, der durch Überführung in ein Aminal, Acetal oder ein gemischtes Aminalacetal geschützt ist, wobei die Aminale, Acetale und gemischten Aminalacetale acyclisch oder cyclisch sein können.

Geschütztes Hydroxy steht im Rahmen der Erfindung für einen Hydroxy-Rest, der durch Überführung in ein Acetal, Carbonat, Carbamat oder Carboxylat geschützt ist. Beispiele dafür sind die Überführung in ein Tetrahydropyranyladdukt, in ein Benzyloxycarbonyl-, Allyloxycarbonyl- oder ein Tert.-Butyloxycarbonyl-Derivat.

Substituiertes oder unsubstituiertes Aryloxy steht beispielsweise und bevorzugt für Reste der Formel (IV)

-O-Ar (IV)

in der Ar die gleiche weiteste Bedeutung besitzt wie sie für substituiertes oder unsubstituiertes Aryl oben angegeben wurde.

Besonders bevorzugt steht unsubstituiertes oder substituiertes Aryloxy für Reste der allgemeinen Formel (IV), in der Ar für Phenyl, Naphtyl, Anthracenyl, Phenanthrenyl, Pyridinyl, Pyrazinyl, Pyridazinyl oder Pyrimidinyl steht, die pro Cyclus mit keinem, einem, zwei oder drei weiteren Substituenten aus der Reihe freies oder geschütztes Hydroxy, Brom, Chlor, Fluor, Cyano, Methyl, Ethyl, n-Propyl, iso-Propyl, n-Butyl, n-Pentyl, n-Hexyl, Phenyl, Benzyl, C₁-C₁₂-Perfluoralkyl wie beispielsweise, Trifluormethyl, Pentafluorethyl, und Substituenten der allgemeinen Formeln (II) und (IIIa) und (IIIb) substituiert sein können, in denen jeweils unabhängig voneinander
- A: fehlt oder für Methylen steht
- B: fehlt oder für Sauerstoff oder NR³ steht,
wobei
R³ Wasserstoff, Methyl oder Ethyl und
D für eine Carbonyl-Gruppe steht und
E für R⁴, OR⁴, NHR⁵ oder NR⁵R⁶ steht,
wobei
R⁴ für Methyl, Ethyl, n-Propyl, iso-Propyl, n-Butyl, tert.-Butyl, Benzyl, 2-Hydroxyethyl, Trifluormethyl oder Phenyl und
R⁵ und R⁶ jeweils unabhängig für Methyl, Ethyl, n-Propyl, iso-Propyl, n-Butyl, Benzyl, 2-Hydroxyethyl oder Phenyl oder
NR⁵R⁶ zusammen für Morpholinyl, Piperidinyl oder Pyrolidinyl stehen und
X für OH, NH₂, oder OM steht, wobei M für ein Natrium-, Kalium- oder Ammoniumion steht.
Ganz besonders bevorzugt steht unsubstituiertes oder substituiertes Aryloxy für Reste der allgemeinen Formel (IV), in der Ar für Phenyl steht, das mit keinem, einem oder zwei weiteren Substituenten substituiert ist, die ausgewählt sind aus der Gruppe Fluor, Chlor, Cyano, Methoxy, Methyl, Ethyl, Phenyl, Trifluormethyl, und Resten der allgemeinen Formeln (II) sowie (IIIa) und (IIIb), in denen
A und B fehlen und
D für eine Carbonyl-Gruppe steht und
E für R⁴ oder OR⁴ steht,
wobei
R⁴ für Methyl, Ethyl oder Phenyl steht.
Noch weiter bevorzugt steht unsubstituiertes oder substituiertes Aryloxy für Phenoxy, 2,4-Dimethylphenoxy, 3,5-Dimethylphenoxy, 3,5-Bis(trifluormethyl)-phenoxy, 4-Methylphenoxy, 3-Methylphenoxy, 3-Methoxyphenoxy, 4-Methoxyphenoxy, 2-Methoxyphenoxy, 2-Methylphenoxy, 2,4-Dichlorphenoxy, 2-Ethoxycarbonylphenyl, 2-Methoxycarbonyl, 2-Acetylphenyl, 4-Acetylphenyl oder 2,6-Dimethylphenoxy.
Tertiäres Amino steht beispielsweise für Alkylarylamino, Dialkylamino oder Diarylamino, bevorzugt für Dialkylamino oder Diarylamino. Auch cyclische Aminoreste sind vom Rahmen der Erfindung mitumfasst.
Bevorzugte Beispiele für tertiäres Amino sind Di(substituiertes oder unsubstituiertes C₁-C₁₂-Alkyl)amino wie zum Beispiel Dimethylamino, Diethylamino, Diisopropylamino, Di-n-butylamino, Di-(R)-Phenylethylamino, Di-(S)-Phenylethylamino, Dibenzylamin und Di(substituiertes oder unsubstituiertes C₆-C₁₀-Aryl)amino, wie zum Beispiel Diphenylamino, Di-(p-Tolyl)amino oder cyclische Aminoreste wie R,R-Dimethylpyrolidino, S,S-Dimethylpyrrolidino, Morpholino, Piperidino, Tetramethylpiperidino.
R¹ steht beispielsweise und bevorzugt für einen unsubstituierten oder substituierten 1,1'-Biphenyl-2,2'-diyl-Rest der allgemeinen Formel (VI), 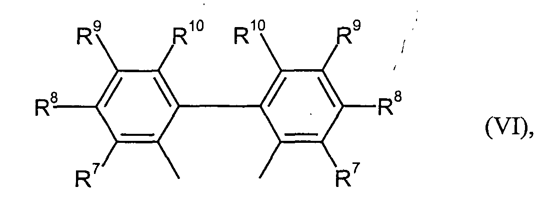 in der die Reste R⁷, R⁸, R⁹und R¹⁰ jeweils unabhängig voneinander ausgewählt sind aus der Gruppe Fluor, Chlor, Brom, unsubstituiertes oder substituiertes geschütztes Hydroxy, unsubstituiertes oder substituiertes C₁-C₆-Alkyl, unsubstituiertes oder substituiertes C₁-C₆-Alkoxy, unsubstituiertes oder substituiertes C₁-C₆-Alkylthio, Cyano, freies oder geschütztes Formyl, unsubstituiertes oder substituiertes C₆-C₁₂-Aryl, Tri(C₁-C₆-alkyl)siloxyl oder Resten der allgemeinen Formel (II),

A-B-D-E (II),

in der unabhängig voneinander
A fehlt oder für einen C₁-C₈-Alkylenrest steht und
B fehlt oder für Sauerstoff, Schwefel oder NR³ steht,
wobei
R³ Wasserstoff, C₁-C₁₆-Alkyl oder C₆-C₁₀-Aryl bedeutet und
D für eine Carbonyl-Gruppe steht und
E für R⁴, OR⁴, NHR⁵ oder NR⁵R⁶ steht,
wobei
R⁴ für C₁-C₁₂-Alkyl oder C₆-C₁₀-Aryl und
R⁵ und R⁶ jeweils unabhängig voneinander für C₁-C₈-Alkyl oder C₆-C₁₀-Aryl stehen oder NR⁵R⁶ zusammen für einen cyclischen Aminorest steht,
oder Resten der allgemeinen Formeln (IIIa) und (IIIb) steht mit der oben angegebenen weitesten Bedeutung steht.

Die beiden Reste R¹⁰ können zusammen auch verbrückend sein. Von der Erfindung sind auch die Fälle umfasst, in denen die beiden Reste R¹⁰ jeweils chiral sind oder zusammen chiral und verbrückend sind.

Weiterhin können auch jeweils zwei Reste einen nicht aromatischen Ring bilden.

R¹ steht besonders bevorzugt für einen unsubstituierten oder substituierten 1,1'-Biphenyl-2,2'-diyl-Rest der allgemeinen Formel (VI), in dem die Reste R⁷, R⁸, R⁹ und R¹⁰ jeweils unabhängig voneinander ausgewählt sind aus der Gruppe Fluor, Chlor, Brom, freies oder geschütztes Hydroxy, unsubstituiertes oder substituiertes C₁-C₄-Alkyl, unsubstituiertes oder substituiertes C₁-C₄-Alkoxy, unsubstituiertes oder substituiertes C₁-C₄-Alkylthio, Cyano, C₆-Aryl, Tri(C₁-C₄-alkyl)siloxyl oder Resten der allgemeinen Formel (II),

A-B-D-E (II)

in der unabhängig voneinander
- A: fehlt oder für einen C₁-C₄-Alkylenrest steht und
- B: fehlt oder für Sauerstoff oder NR³ steht,
wobei R³ Wasserstoff, C₁-C₆-Alkyl oder C₆-C₁₀Aryl bedeutet und
- D: für eine Carbonyl-Gruppe steht und
- E: für R⁴, OR⁴, NHR⁵ oder NR⁵R⁶ steht,
wobei R⁴ für C₁-C₆-Alkyl oder C₆-C₁₀ Aryl und
R⁵ und R⁶ jeweils unabhängig voneinander für C₁-C₄-Alkyl oder C₆-Aryl stehen oder NR⁵R⁶ zusammen für einen cyclischen Aminorest steht,
oder Resten der allgemeinen Formeln (IIIa) und (IIIb) mit der oben angegebenen weitesten Bedeutung steht.

Die beiden Reste R¹⁰ können zusammen auch verbrückend sein. Hier kommen bevorzugt und beispielsweise Verbrückungen der Formel (VII) in Betracht

-O-G¹-K-G²-O- (VII)

in denen unabhängig voneinander G¹ und G² entweder entfallen kann, eine Carbonylgruppe oder eine Carbonylaminogruppe sein kann,

K eine unsubstituierte oder substituierte C₂-C₆-Alkylenkette sein kann.

R¹ steht ganz besonders bevorzugt für einen unsubstituierten oder substituierten 1,1'-Biphenyl-2,2'-diyl-Rest der allgemeinen Formel (VI), in der die Reste R⁷, R⁸, R⁹ und R¹⁰ jeweils unabhängig voneinander ausgewählt sind aus der Gruppe Fluor, Chlor, Brom, freies oder geschütztes Hydroxy, unsubstituiertes oder substituiertes C₁-C₄-Alkyl, unsubstituiertes oder substituiertes C₁-C₄-Alkoxy, oder Resten der allgemeinen Formel (II),

A-B-D-E (II)

in der unabhängig voneinander
A fehlt und
B fehlt oder für Sauerstoff steht,
und
D für eine Carbonyl-Gruppe steht und
E für R⁴, OR⁴, NHR⁵ oder NR⁵R⁶ steht,
wobei R⁴ für C₁-C₄-Alkyl oder C₆-C₁₀-Aryl und
R⁵ und R⁶ jeweils unabhängig voneinander für C₁-C₄-Alkyl stehen oder NR⁵R⁶ zusammen für einen cyclischen Aminorest steht,
oder Resten der allgemeinen Formeln (IIIa) und (IIIb) mit der oben angegebenen weitesten Bedeutung steht oder die beiden Reste R¹⁰ für Verbrückungen der allgemeinen Formel (VII) stehen, in der G entweder entfallen kann, eine Carbonylgruppe oder eine Carbonylaminogruppe ist und

K für eine unsubstituierte oder substituierte C₂ - C₄ - Alkylenkette steht.

Noch weiter bevorzugt steht R¹ für folgende Reste: 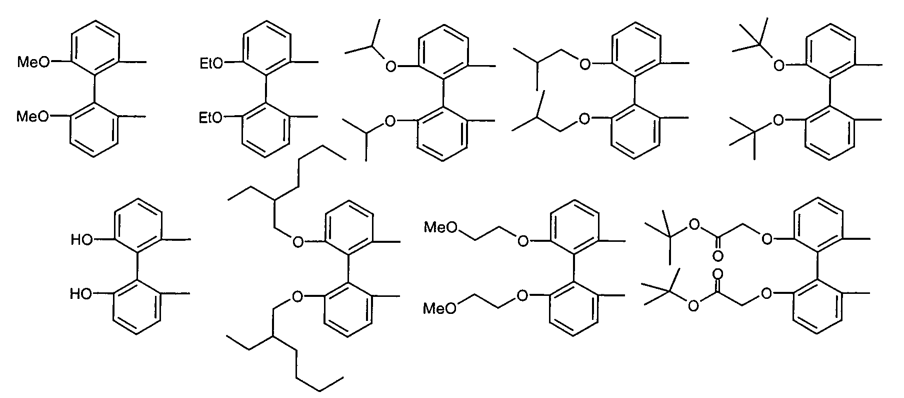 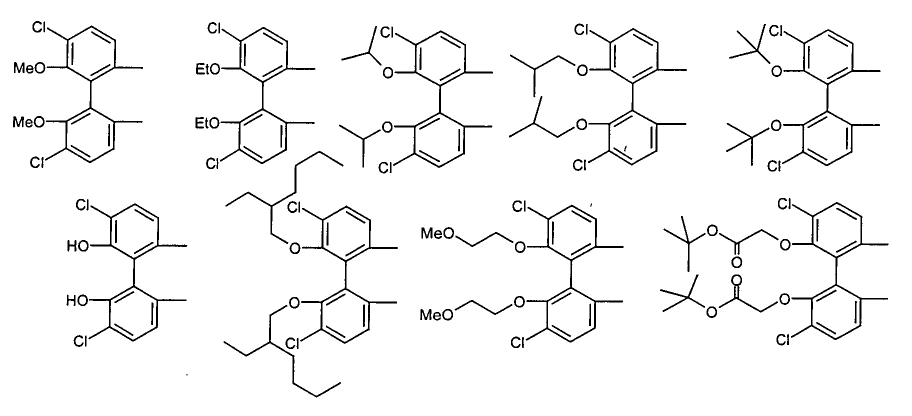 oder für solche Reste der allgemeinen Formel (VIII) 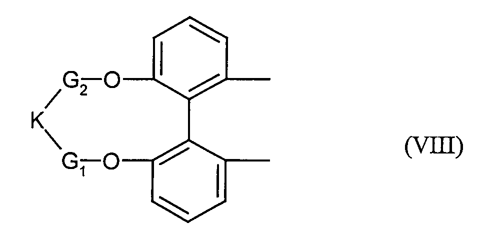 in der G¹-K-G² zusammen für (R)-1,2-Propandiyl, (S,S)-1,2-Cyclohexandiyl, (R,R)-1,2-Cyclohexandiyl, 1,2-Ethandiyl, 1,3-Propandiyl, 1,4-Butandiyl oder 1,4-Dioxobutandiyl steht oder für folgende Reste:

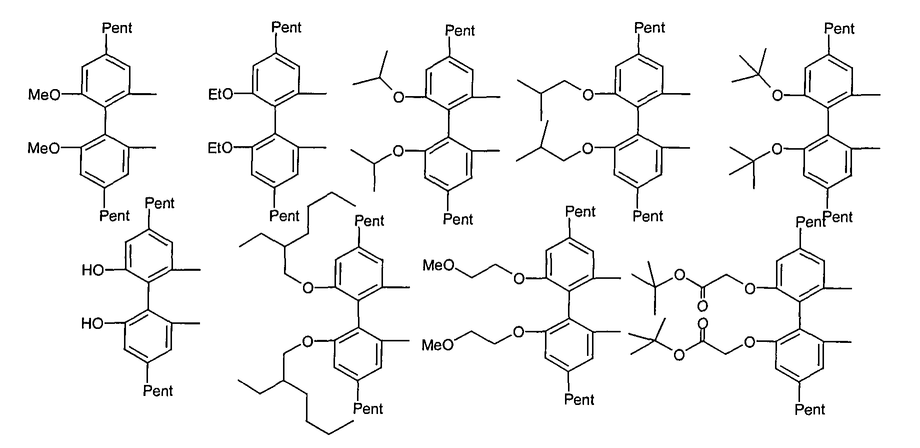

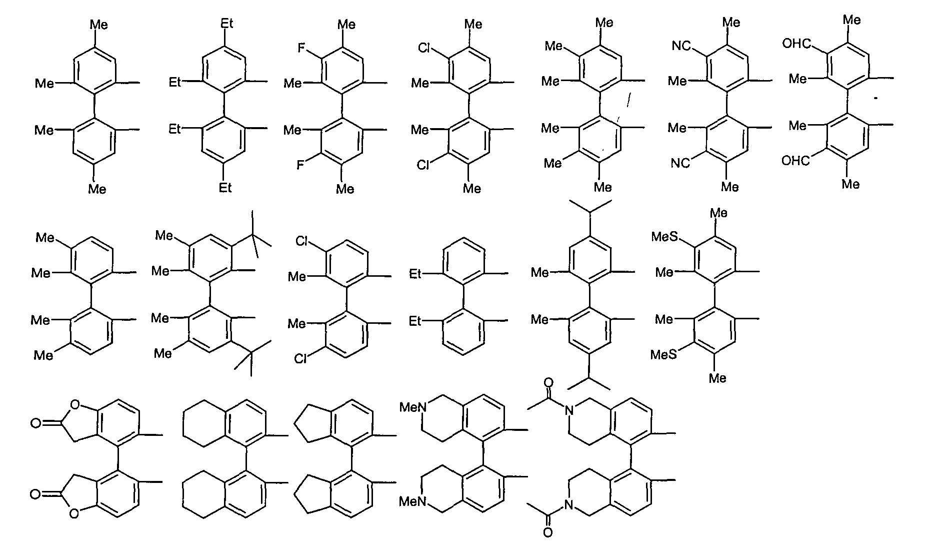

Es sei an dieser Stelle darauf hingewiesen, dass die Vorzugsbereiche der unter R² genannten Reste in beliebiger Kombination mit den Vorzugsbereichen von R¹ vom Rahmen der Erfindung mitumfasst sind.

Von der Erfindung sind alle stereoisomeren Verbindungen der chiralen Monophosphorverbindungen der allgemeinen Formel (I) umfasst, und zwar sowohl in reiner Form als auch in Form von beliebigen Mischungen von stereoisomeren Verbindungen, wie zum Beispiel Racemate oder Diastereomerengemische.

Als bevorzugte Verbindungen der allgemeinen Formel (I) seien genannt:
((S)-5,5'-Dichlor-6,6'-dimethoxy-1,1'-biphenyl-2,2'-diyl)-isopropyl-phosphit
((R)-5,5'-Dichlor-6,6'-dimethoxy-1,1'-biphenyl-2,2'-diyl)-isopropyl-phosphit
((R)-5,5'-Dichlor-6,6'-dimethoxy-1,1'-biphenyl-2,2'-diyl)-(R)-1-phenylethylphosphit
((R)-5,5'-Dichlor-6,6'-dimethoxy-1,1'-biphenyl-2,2'-diyl)-(S)-1-phenylethylphosphit
((S)-5,5'-Dichlor-6,6'-dimethoxy-1,1'-biphenyl-2,2'-diyl)-(R)-1-phenylethylphosphit
((S)-5,5'-Dichlor-6,6'-dimethoxy-1,1'-biphenyl-2,2'-diyl)-(S)-1-phenylethylphosphit
((S)-5,5 '-Dichlor-6,6'-dimethoxy-1,1'-biphenyl-2,2'-diyl)-cyclohexyl-phosphit
((R)-5,5'-Dichlor-6,6'-dimethoxy-1,1'-biphenyl-2,2'-diyl)-cyclohexyl-phosphit
((S)-5,5'-Dichlor-6,6'-dimethoxy-1,1'-biphenyl-2,2'-diyl)-phenyl-phosphit
((R)-5,5'-Dichlor-6,6'-dimethoxy-1,1'-biphenyl-2,2'-diyl)-phenyl-phosphit
((S)-5,5'-Dichlor-6,6'-dimethoxy-1,1'-biphenyl-2,2'-diyl)-2,6-dimethylphenylphosphit
((R)-5,5'-Dichlor-6,6'-dimethoxy-1,1'-biphenyl-2,2'-diyl)-2,6-dimethylphenylphosphit
((S)-5,5',6,6'-tetramethyl-3,3'-bis(tert.-butyl)-1,1'-biphenyl-2,2'-diyl)-isopropylphosphit
((R)-5,5',6,6'-tetramethyl-3,3 '-bis(tert.-butyl)-1,1'-biphenyl-2,2'-diyl)-isopropylphosphit
((S)-5,5',6,6'-tetramethyl-3,3'-bis(tert.-butyl)-1,1'-biphenyl-2,2'-diyl)-(rac)-1-phenylethyl-phosphit
((R)-5,5',6,6'-tetramethyl-3,3'-bis(tert.-butyl)-1,1'-biphenyl-2,2'-diyl)-(rac)-1-phenylethyl-phosphit
((S)-5,5',6,6'-tetramethyl-3,3'-bis(tert.-butyl)-1,1'-biphenyl-2,2'-diyl)-(S)-1-phenylethyl-phosphit
((R)-5,5',6,6'-tetramethyl-3,3'-bis(tert.-butyl)-1,1'-biphenyl-2,2'-diyl)-(S)-1-phenylethyl-phosphit
((S)-5,5',6,6'-tetramethyl-3,3'-bis(tert.-butyl)-1,1'-biphenyl-2,2'-diyl)-(R)-1-phenylethyl-phosphit
((R)-5,5',6,6'-tetramethyl-3,3 '-bis(tert.-butyl)-1,1'-biphenyl-2,2'-diyl)-(R)-1-phenylethyl-phosphit
((S)-5,5',6,6'-tetramethyl-3,3'-bis(tert.-butyl)-1,1'-biphenyl-2,2'-diyl)-diphenylmethyl-phosphit
((R)-5,5',6,6'-tetramethyl-3,3'-bis(tert.-butyl)-1,1'-biphenyl-2,2'-diyl)-diphenylmethyl-phosphit
((S)-5,5',6,6'-tetramethyl-3,3'-bis(tert.-butyl)-1,1'-biphenyl-2,2'-diyl)-methyl-phosphit
((R)-5,5',6,6'-tetramethyl-3,3 '-bis(tert.-butyl)-1,1'-biphenyl-2,2'-diyl)-methyl-phosphit
((S)-5,5',6,6'-tetramethyl-3,3 '-bis(tert.-butyl)-1,1'-biphenyl-2,2'-diyl)-2,6-dimethylphenyl-phosphit
((R)-5,5',6,6'-tetramethyl-3,3'-bis(tert.-butyl)-1,1'-biphenyl-2,2'-diyl)-2,6-dimethylphenyl-phosphit
((S)-5,5',6,6'-tetramethyl-3,3'-bis(tert.-butyl)-1,1 '-biphenyl-2,2'-diyl)-2,6-diisopropylphenyl-phosphit
((R)-5,5',6,6'-tetramethyl-3,3'-bis(tert.-butyl)-1,1 '-biphenyl-2,2'-diyl)-2,6-diisopropylphenyl-phosphit
((S)-5,5',6,6'-tetramethyl-3,3'-bis(tert.-butyl)-1,1'-biphenyl-2,2'-diyl)-phenylphosphit
((R)-5,5',6,6'-tetramethyl-3,3'-bis(tert.-butyl)-1,1'-biphenyl-2,2'-diyl)-phenylphosphit
((S)-5,5',6,6'-tetramethyl-3,3'-bis(tert.-butyl)-1,1'-biphenyl-2,2'-diyl)-ethyl-phosphit
((R)-5,5',6,6'-tetramethyl-3,3'-bis(tert.-butyl)-1,1'-biphenyl-2,2'-diyl)-ethyl-phosphit
((S)-5,5',6,6'-tetramethyl-3,3'-bis(tert.-butyl)-1,1'-biphenyl-2,2'-diyl)-4-tert-butylphenyl-phosphit
((R)-5,5',6,6'-tetramethyl-3,3'-bis(tert.-butyl)-1,1'-biphenyl-2,2'-diyl)-4-tert-butylphenyl-phosphit

Die erfindungsgemäßen chiralen Monophosphorverbindungen sind in an sich bekannter Weise herstellbar. Die Herstellung kann beispielsweise erfolgen, indem
- Diole der allgemeinen Formel (IX),

   HO-R¹-OH (IX)

   in der R¹ die unter der allgemeinen Formel (I) angegebene Bedeutung besitzt,
- mit aktivierten Phosphanen der allgemeinen Formel (X)

   (Akt)ₙP(R²)₃₋ₙ (X)

   wobei
   - Akt: für Chlor, Brom, Iod, Dialkylamino wie beispielsweise Dimethylamino oder Diethylamino steht und
   - R²: die unter der allgemeinen Formel (I) angegebene Bedeutung besitzt und
   - n: für 2 oder 3 steht
- in Gegenwart einer Base wie zum Beispiel Triethylamin oder nach Deprotonierung des eingesetzten Diols der allgemeinen Formel (IX) miteinander umsetzt.
Bevorzugt steht
Akt für Chlor oder Dimethylamino oder Diethylamino,
besonders bevorzugt für Chlor.

Ist n = 3, so entstehen zunächst als Zwischenprodukte Verbindungen der allgemeinen Formel (XI) 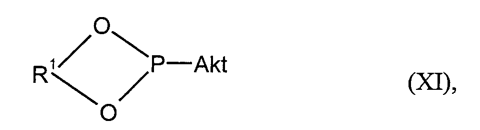 die in einem weiteren Schritt mit einer Verbindung der allgemeinen Formel (XII)

H-R¹¹ (XII),

in der R¹¹ für einen Rest steht, der ausgewählt ist aus der Gruppe substituiertes oder unsubstituiertes Alkoxy, substituiertes oder unsubstituiertes Aryloxy, sekundäres oder tertiäres Amino und wobei die gleiche Definition gilt, die für die genannten Reste unter R¹ angegeben wurde,
gegebenenfalls in Gegenwart einer Base wie zum Beispiel Triethylamin oder nach vorheriger Deprotonierung zu den chiralen Monophosphorverbindungen der allgemeinen Formel (I) umgesetzt werden können.

Die Verbindungen der allgemeinen Formel (XI) sind ebenfalls von der Erfindung umfasst.

Die Trennung von Stereoisomeren kann beispielsweise dadurch erfolgen, dass Biphenylverbindungen der allgemeinen Formel (VI) durch Co-Kristallisation mit geeigneten chiralen, enantiomerenangereicherten Hilfsstoffen, wie zum Beispiel chiralen enantiomerenreinen Aminen, in die Enantiomeren getrennt werden. Die Herstellung enantiomerenreiner Biphenole der allgemeinen Formel (VI) kann ebenso dadurch erfolgen, dass zunächst mit einer geeigneten aktivierten Phosphorverbindung wie zum Beispiel PCl₃ oder P(NMe₂)₃ umgesetzt wird, (vgl. K. Sasse, Methoden der Organischen Chemie, Houben-Weyl, Georg Thieme Verlag, 1964, Vol. XII/2,4. Aufl., 5-130) und nach weiterer Umsetzung mit einem enantiomerenreinen Alkohol, wie zum Beispiel Menthol, diastereomere Phosphite erzeugt werden, die in üblicher Weise getrennt und nach anschließender Spaltung zu enantiomerenangereicherten Biphenolen der allgemeinen Formel (VI) führen. Weiterhin sind Verbindungen der Formel (VI) sowie der Formel (I) durch Chromatographie an chiralen stationären Phasen in ihre Enantiomere auftrennbar. Weiterhin können enantiomerenreine Biphenylverbindungen der Formel (VI) durch Umsetzung mit enantiomerenreinen Biselektrophilen und substituierten 2,2',6,6'-Tetrahydroxy-1,1'-biphenylen in Analogie zu T. Harada et al. (Organic Letters, 2000, Vol.2, p. 1319) erhalten werden.

Die Erfindung schließt auch Katalysatoren ein, die Übergangsmetallkomplexe der erfindungsgemäßen chiralen Monophosphorverbindungen der allgemeinen Formel (I) enthalten. Insbesondere sind dies Übergangsmetallkomplexe von Ruthenium, Osmium, Cobalt, Rhodium, Iridium, Nickel, Palladium, Platin und Kupfer, bevorzugt solche von Ruthenium, Rhodium, Iridium, Nickel, Palladium, Platin und Kupfer.

Als Katalysatoren können beispielsweise entweder isolierte Übergangsmetallkomplexe eingesetzt werden oder Übergangsmetallkomplexe, die aus den chiralen Monophosphorverbindungen der allgemeinen Formel (I) und einer Metallverbindung erzeugt werden.

Bevorzugt werden als Katalysatoren Übergangsmetallkomplexe, die aus chiralen Monophosphorverbindungen der allgemeinen Formel (I) und mindestens einer Metallverbindung erzeugt werden, eingesetzt.

Geeignete Metallverbindungen sind beispielsweise und bevorzugt solche der allgemeinen Formel

M(Y¹)ₚ (XIIIa),

in der
- M: für Ruthenium, Rhodium, Iridium, Nickel, Palladium, Platin oder Kupfer und
- Y¹: für Chlorid, Bromid, Acetat, Nitrat, Methansulfonat, Trifluormethansulfonat oder Acetylacetonat und
- p: für Ruthenium, Rhodium und Iridium für 3, für
Nickel, Palladium und Platin für 2 und für
Kupfer für 1 stehen,
oder Metallverbindungen der allgemeinen Formel (XIIIb)

M(Y²)ₚB¹ ₂ (XIIIb),

in der
- M: für Ruthenium, Rhodium, Iridium, Nickel, Palladium, Platin oder Kupfer und
- Y²: für Chlorid, Bromid, Acetat, Methansulfonat, Trifluormethansulfonat, Tetrafluoroborat, Hexafluorophosphat Perchlorat, Hexafluoroantimonat, Tetra(bis-3,5-trifluormethylphenyl)borat oder Tetraphenylborat stehen und
- p: für Rhodium und Iridium für 1, für
Nickel, Palladium, Platin und Ruthenium für 2 und für
Kupfer für 1 steht,
- B¹: jeweils für ein C₂-C₁₂-Alken wie beispielsweise Ethylen oder Cycloocten, oder ein Nitril wie beispielsweise Acetonitril, Benzonitril oder Benzylnitril steht, oder
- B¹₂: zusammen für ein (C₄-C₁₂)-Dien wie beispielsweise Norbornadien oder 1,5-Cyclooctadien steht
oder Metallverbindungen der allgemeinen Formel (XIIIc)

[MB²Y¹ ₂]₂ (XIIIc),

in der
M für Ruthenium und
- B²: für Arylreste wie zum Beispiel Cymol, Mesityl, Phenyl oder Cyclooctadien, Norbornadien oder Methylallyl stehen
oder Metallverbindungen der allgemeinen Formel (XIIId)

Meₚ[M(Y³)₄] (XIIId),

wobei
- M: für Palladium, Nickel, Iridium oder Rhodium und
- Y³: für Chlorid oder Bromid stehen und
- Me: für Lithium, Natrium, Kalium, Ammonium oder organisches Ammonium steht und
- p: für Rhodium und Iridium für 3, für
Nickel, Palladium und Platin für 2 steht,
oder Metallverbindungen der allgemeinen Formel (XIIIe)

[M(B³)₂]An (XIIIe),

wobei
- M: für Iridium oder Rhodium und
- B³: für ein (C₄-C₁₂)-Dien wie beispielsweise Norbornadien oder 1,5-Cyclooctadien stehen
- An: für ein nicht oder schwach koordinierendes Anion wie zum Beispiel Methansulfonat, Trifluormethansulfonat, Tetrafluoroborat, Hexafluorophosphat Perchlorat, Hexafluoroantimonat, Tetra(bis-3,5-trifluormethylphenyl)borat oder Tetraphenylborat steht.

Darüber hinaus sind als Metallverbindungen beispielsweise Ni(1,5-Cyclooctadien)₂, Pd₂(dibenzylidenaceton)₃, Pd[PPh₃]₄ Cyclopentadienyl₂Ru, Rh(acac)(CO)₂, Ir(pyridin)₂(1,5-Cyclooctadien), Cu(Phenyl)Br, Cu(Phenyl)Cl, Cu(Phenyl)I, Cu(PPh₃)₂Br, [Cu(CH₃CN)₄]BF₄ und [Cu(CH₃CN)₄]PF₆ oder mehrkernige verbrückte Komplexe wie beispielsweise [Rh(1,5-cyclooctadien)Cl]₂ und [Rh(1,5-cyclooctadien)Br]₂, [Rh(Ethen)₂Cl]₂, [Rh(Cycloocten)₂Cl]₂ geeignet.

Bevorzugt werden als Metallverbindungen eingesetzt:
[Rh(COD)Cl]₂, [Rh(COD)₂Br], [Rh(COD)₂]ClO₄, [Rh(COD)₂]BF₄, [Rh(COD)₂]PF₆, [Rh(COD)₂]OTf, [Rh(COD)₂]BAr₄ (Ar = 3,5-bistrifluormethylphenyl) [Rh(COD)₂]SbF₆ RuCl₂(COD), [(Cymol)RuCl₂]₂, [(Benzol)RuCl₂]_{2,} [(Mesityl)RuCl₂]₂, [(Cymol)RuBr₂]_{2,} [(Cymol)RuI₂]_{2,} [(Cymol)Ru(BF₄)₂]₂, [(Cymol)Ru(PF₆)₂]₂, [(Cymol)Ru(BAr₄)₂]₂, (Ar = 3,5-bistrifluormethylphenyl), [(Cymol)Ru(SbF₆)₂]₂, [Ir(cod)₂Cl]₂, [Ir(COD)₂]PF₆, [Ir(COD)₂]ClO₄, [Ir(COD)₂]SbF₆ [Ir(COD)₂]BF₄, [Ir(COD)₂]OTf, [Ir(COD)₂]BAr₄ (Ar = 3,5-bistrifluormethylphenyl) RuCl₃, NiCl₂, RhCl₃, PdCl₂, PdBr₂, Pd(OAc)₂, Pd₂(dibenzylidenaceton)₃, Pd(acetylacetonat)₂, CuOTf, CuI, CuCl, Cu(OTf)₂, CuBr, CuI, CuBr₂, CuCl₂, CuI₂,
[Rh(nbd)Cl]₂, [Rh(nbd)₂Br], [Rh(nbd)₂]ClO₄, [Rh(nbd)₂]BF₄, [Rh(nbd)₂]PF₆, [Rh(nbd)₂]OTf, [Rh(nbd)₂]BAr₄ (Ar = 3,5-bistrifluormethylphenyl) [Rh(nbd)₂]SbF₆ RuCl₂(nbd), [Ir(nbd)₂]PF₆, [Ir(nbd)₂]ClO₄, [Ir(nbd)₂]SbF₆ [Ir(nbd)₂]BF_{4,} [Ir(nbd)₂]OTf, [Ir(nbd)₂]BAr₄ (Ar = 3,5-bistrifluormethylphenyl), Ir(pyridin)₂(nbd), [Ru(DMSO)₄Cl₂], [Ru(CH₃CN)₄Cl₂], [Ru(PhCN)₄Cl₂], [Ru(COD)Cl₂]ₙ, [Ru(COD)(Methallyl)₂], [Ru(acetylacetonat)₃]

Noch weiter bevorzugt sind Rh(COD)₂-trifluormethansulfonat, Rh(nbd)₂PF₆ und Rh(nbd)₂BF₄.

Die Menge der eingesetzten Metallverbindung kann beispielsweise 25 bis 200 mol-% bezogen auf die eingesetzte chirale Monophosphorverbindung der allgemeinen Formel (I) betragen, bevorzugt sind 30 bis 100 mol-%, ganz besonders bevorzugt 40 bis 60 mol-% und noch weiter bevorzugt 45 bis 55 mol-% .

Die Katalysatoren, die in situ erzeugte Übergangsmetallkomplexe oder isolierte Übergangsmetallkomplexe enthalten, eignen sich insbesondere für den Einsatz in einem Verfahren zur Herstellung von chiralen Verbindungen.

Bevorzugt werden die Katalysatoren für asymmetrische 1,4-Additionen, asymmetrische Hydroformylierungen, asymmetrische Hydrocyanierungen, asymmetrische Heck-Reaktionen und asymmetrische Hydrogenierungen eingesetzt, besonders bevorzugt für asymmetrische Hydrogenierungen.

Bevorzugte asymmetrische Hydrogenierungen sind beispielsweise Hydrogenierungen von prochiralen C=C-Bindungen wie zum Beispiel prochirale Enamine, Olefine, Enolether, C=O-Bindungen wie zum Beispiel prochirale Ketone und C=N-Bindungen wie zum Beispiel prochirale Imine. Besonders bevorzugte asymmetrische Hydrogenierungen sind Hydrogenierungen von prochiralen Enaminen und Olefinen.

Die Menge der eingesetzten Metallverbindung oder des eingesetzten Übergangsmetallkomplexes kann beispielsweise 0,001 bis 5 mol-% bezogen auf das eingesetzte Substrat betragen, bevorzugt sind 0,001 bis 0,5 mol-%, ganz besonders bevorzugt 0.001 bis 0,1 mol-% und noch weiter bevorzugt 0,001 bis 0,008 mol-% .

In einer bevorzugten Ausführungsform können asymmetrische Hydrogenierungen beispielsweise so durchgeführt werden, dass der Katalysator in situ aus einer Metallverbindung und einer chiralen Monophosphorverbindung der allgemeinen Formel (I) gegebenenfalls in einem geeigneten Lösungsmittel erzeugt wird, das Substrat zugegeben wird und die Reaktionsmischung bei Reaktionstemperatur unter Wasserstoffdruck gesetzt wird.

Bevorzugt kommen für asymmetrische Hydrogenierungen als Metallverbindungen solche der allgemeinen Formel (XIIIe) zum Einsatz

[M(B³)₂]An (XIIIe),

wobei
- M: für Rhodium und
- B³: für ein (C₄-C₁₂)-Dien wie beispielsweise Norbornadien oder 1,5-Cyclooctadien stehen und
- An: für ein nicht oder schwach koordinierendes Anion wie zum Beispiel Methansulfonat, Trifluormethansulfonat, Tetrafluoroborat, Hexafluoro-phosphat Perchlorat, Hexafluoroantimonat, Hexachloroantimonat, Tetra(bis-3,5-trifluormethylphenyl)borat oder Tetraphenylborat steht oder
zweikernige Komplexe wie zum Beispiel [Rh(1,5-cyclooctadien)Cl]₂ und [Rh(1,5-cyclooctadien)Br]₂, [Rh(Ethen)₂Cl]₂, [Rh(Cycloocten)₂Cl]₂

Besonders bevorzugte Metallverbindungen für asymmetrische Hydrogenierungen sind [Rh(1,5-cyclooctadien)₂]BF₄, [Rh(1,5-cyclooctadien)₂]PF₆, [Rh(Norbornadien)₂]PF₆ und [Rh(Norbornadien)₂]BF₄.

In einer besonders bevorzugten Ausführungsform werden in einem ausgeheizten Glasautoklaven Metallverbindung und Monophosphorverbindung in entgastem Lösungsmittel gelöst. Man lässt ca. 5 min rühren und gibt anschließend das Substrat in entgastem Lösungsmittel zu. Nach dem Einstellen der jeweiligen Temperatur wird mit H₂-Überdruck hydriert.

Als Lösungsmittel für die asymmetrische Hydrogenierung eignen sich beispielsweise chlorierte Alkane wie Methylenchlorid, kurzkettige C₁-C₆-Alkohole wie z.B. Methanol, iso-Propanol oder Ethanol, aromatische Kohlenwasserstoffe wie z.B. Toluol oder Benzol, Ketone wie z.B. Aceton oder Carbonsäureester wie z.B. Ethylacetat.

Die asymmetrische Hydrogenierung wird beispielsweise bei einer Temperatur von -20°C bis 200°C, bevorzugt 0 bis 100°C und besonders bevorzugt bei 20 bis 70°C durchgeführt.

Der Wasserstoffdruck kann beispielsweise 0,1 bis 200 bar, bevorzugt 0,5 bis 50 und besonders bevorzugt 0,5 bis 5 bar betragen.

Die erfindungsgemäßen Katalysatoren eignen sich insbesondere in einem Verfahren zur Herstellung von chiralen Wirkstoffen in Arzneimitteln und Agrarchemikalien, oder Zwischenprodukten dieser beiden Klassen.

Der Vorteil der vorliegenden Erfindung ist, dass mit einfach herzustellenden Liganden insbesondere in asymmetrischen Hydrogenierungen bislang unerreichte Aktivitäten von weit über 1000 h⁻¹ (TOF) erreicht werden können.

### Beispiele:

### Beispiel 1: Synthese von (S)-5,5'-Dichlor-6,6'-dimethoxy-1,1'-biphenyl-2,2'dioxy)-chlor-phosphan

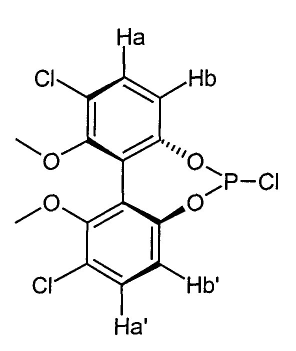

Zu einer Mischung aus 0,41 ml (4,12 mmol) PCl₃ und 0,97 ml (6,98 mmol) NEt₃ in 5 ml THF wird unter Eiskühlung eine Lösung von 1 g (3,17 mmol) (S)-Cl-MeO-Biphenol getropft. Man lässt 1 h bei RT rühren, filtriert den gebildeten Niederschlag ab und wäscht mit wenig Lösungsmittel nach. Nach dem Entfernen des Lösungsmittels erhält man das Produkt als gelbliches Öl.
¹H-NMR (CDCl₃): δ [ppm] = 3.57 (s, 3H, OCH₃); 3.59 (s, 3H, OCH₃); 6.92 (d, ³J = 8.7, 1H, H_{b o. b'}); 7.01 (dd, 1H, ³J = 8.7, J(H-P) = 1.1, 1H, H_{b o. b'}); 7,46 (br d, ³J = 8.7, 2H, H_{a u. a'}); ³¹P-NMR (CDCl₃):
δ [ppm] = 176.4

### Beispiele 2-6: Synthese von (S)-Phosphiten

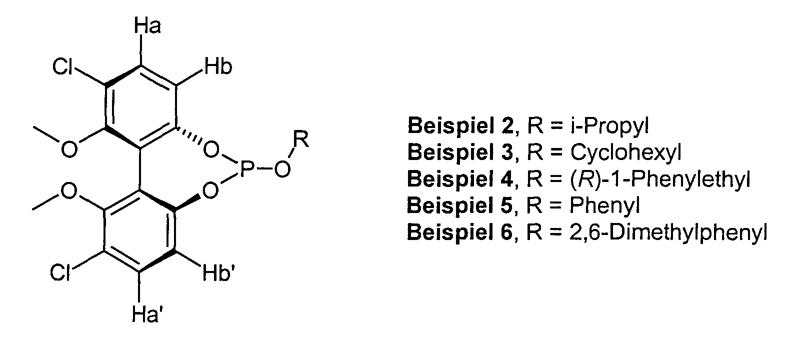

3.17 mmol des entsprechenden Alkohols (i-Propanol, Cyclohexanol, (R)-1-Phenylethanol, Phenol, 2,6-Dimethylphenol) werden in 5 ml THF gelöst und mit 0,44 ml (3,17 mmol) NEt₃ versetzt. 1,204 g (3,17 mmol) Phosphochloridit aus Beispiel 1, gelöst in 10 ml THF, werden bei 0°C zugetropft. Nach 1 h wird der gebildete Niederschlag abfiltriert und mit wenig THF nachgewaschen. Nach Entfernung des Lösungsmittels erhält man die Phosphite als weiße bis leicht gelbliche Feststoffe oder Öle.

### Beispiel 2

### ((S)-5,5'-Dichlor-6,6'-dimethoxy-1,1'-biphenyl-2,2'-diyl)-isopropyl-phosphit

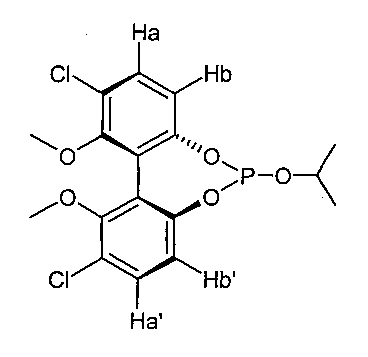

¹H-NMR (CDCl₃): δ [ppm] = 1.29 (d, ³J = 6.2, 3H, CH₃), 1.33 (d, ³J = 6.2, 3H, CH₃); 3.53 u. 3.54 (s, 3H, OCH₃); 4.53 (dsep, ³J = 6.2, J(H-P) = 9.0, 1H, CH); 6.83 (dd, ³J = 8.7, J(H-P) = 0.8, 1H, H_{b o. b'}); 6.96 (dd, ³J = 8.7, J(H-P) = 1.2, 1H, H_{b o. b'}); 7.37 (d, ³J = 8.7, 1H, H_{a o. a'}); 7.40 (dd, ³J = 8.7, J(H-P) = 0.5, 1H, H_{a o. a'}); ³¹P-NMR (CDCl₃):
δ [ppm] = 145.3

### Beispiel 3

### ((S)-5,5'-Dichlor-6,6'-dimethoxy-1,1'-biphenyl-2,2'-diyl)-cyclohexyl-phosphit

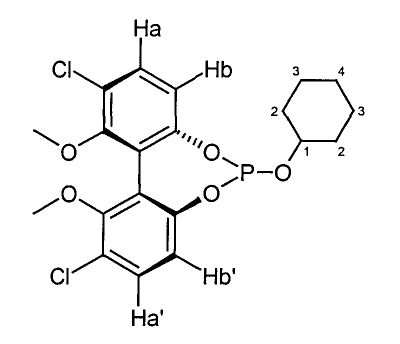

¹H-NMR (CDCl₃): δ [ppm] = 1.00-2.00 (kB, 10H, CH₂); 3.53 u. 3.54 (s, 3H, OCH₃); 4.19 (dsep, J = 4.5, J(H-P) = 9.1, 1H, CH); 6.83 (dd, ³J = 8.7, J(H-P) = 0.7, 1H, H_{b o.} _{b'}); 6.96 (dd, ³J = 8.7, J(H-P) = 1.1, 1H, H_{b o. b'}); 7.36 (d, ³J = 8.7, 1H, H_{a o. a'}); 7.40 (d, ³J = 8.7, 1H, H_{a o. a'}); ³¹P-NMR(CDCl₃): δ [ppm] = 146.1

### Beispiel 4

### ((S)-5,5'-Dichlor-6,6'-dimethoxy-1,1'-biphenyl-2,2'-diyl)-(R)-1-phenylethylphosphit

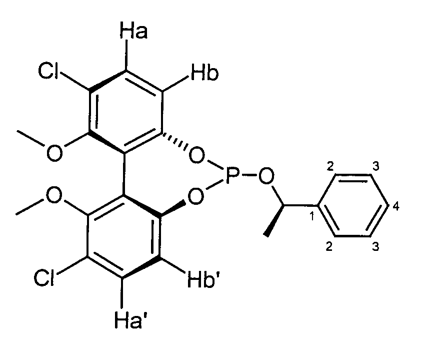

¹H-NMR(CDCl₃): δ [ppm] = 1.59 (d, ³J = 6.5, 3H, CH₃); 3.49 u. 3.53 (s, 3H, OCH₃); 5.38 (dq, ³J = 6.5, J(H-P) = 9.4, 1H, CH); 6.17 (dd, ³J = 8.7, J(H-P) = 0.7, 1H, H_{b o. b'}); 6.95 (dd, ³J = 8.7, J(H-P) = 1.1, 1H, H_{b o. b'}); 7.21 (d, ³J = 8.7, 1H, H_{a o. a'}); 7.25-7.38 (kB, 5H, Hₐᵣₒₘ); 7.38 (d, ³J = 8.7, 1H, H_{a o. a'}); ³¹P-NMR (CDCl₃): δ [ppm] = 146.9

### Beispiel 5:

### ((S)-5,5'-Dichlor-6,6'-dimethoxy-1,1'-biphenyl-2,2'-diyl)-phenyl-phosphit

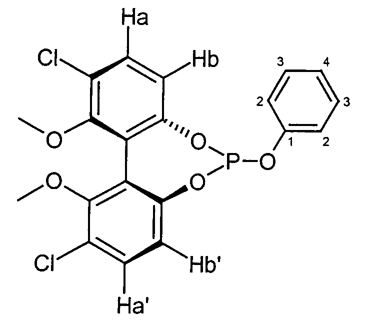

¹H-NMR (CDCl₃): δ [ppm] = 3.59 u. 3.61 (s, 3H, OCH₃); 6.90 (dd, ³J = 8.7, J(H-P) = 0.6, 1H, H_{b o. b'}); 7.06 (dd, ³J = 8.7, J(H-P) = 1.1, 1H, H_{b o. b'}); 7.13-7.21 (kB, 3H, H-2 u. H-4); 7.32-7.38 (kB, 2H, H-3); 7.40 (d, ³J = 8.7, 1H, H_{a o. a'}); 7.47 (d, ³J = 8.7, 1H, H_{a o. a'}); ³¹P-NMR (CDCl₃): δ [ppm] = 141.4

### Beispiel 6:

### ((S)-5,5'-Dichlor-6,6'-dimethoxy-1,1'-biphenyl-2,2'-diyl)-(2,6-dimethylphenyl)-phosphit

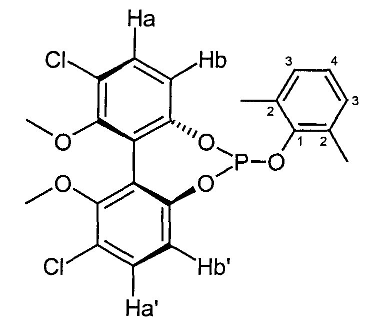

¹H-NMR (CDCl₃): δ [ppm] = 2.40 (s, 6H, CH₃), 3.61 (s, 6H, OCH₃); 7.02 (dd, ³J = 8.7, J(H-P) = 1.0, 1H, H_{b o. b'}); 7.02-7.12 (kB, 3H, Hₐᵣₒₘ); 7.03 (dd, ³J = 8.7, J(H-P) = 0.8, 1H, H_{b o. b'}); 7.45 (d, ³J = 8.7, 1H, H_{a o. a'}); 7.46 (d, ³J = 8.7, 1H, H_{a o. a'}); ³¹P-NMR (CDCl₃): δ [ppm] = 145.1

### Hydrierungen

### Beispiele 7-11

### Hydrierung von Dimethylitakonat

In einem ausgeheizten Glasautoklaven werden 0,02 mmol Bis-(Norbomadien)-Rhodium(I)-Tetrafluoroborat [Rh(nbd)₂]BF₄ und 0,04 mmol des entsprechenden Phosphits in 5 ml entgastem Methylenchlorid gelöst. Man lässt ca. 5 min rühren und gibt anschließend 8 mmol Dimethylitakonat und 0,2000 g Diglyme in 15 ml entgastem Methylenchlorid zu. Nach dem Einstellen der jeweiligen Temperatur wird mit 0,5 bar Wasserstoffpartialdruck 2 h hydriert. Umsatz und ee werden gaschromatographisch bestimmt.

Die Ergebnisse sind in Tabelle 1 zusammengefasst.

**Tabelle 1**

| Beispiel | Ligand (Konfiguration) | Umsatz [%] | | ee [%], Konfigaration | |
|---|---|---|---|---|---|
| | | RT | 0°C | RT | 0°C |
| 7 | R = i-Propyl (*S*) aus Beispiel 2 | 100 | 100 | 96 (S) | 97 (S) |
| 8 | R = Cyclohexyl *(S)* ausBeispiel 3 | 100 | 100 | 91 (*S*) | 94 (*S*) |
| 9 | R = (*R*)-1-Phenylethyl *(S)* aus Beispiel 4 | 100 | 100 | 97 (*S*) | 99 (*S*) |
| 10 | R = Phenyl (*S*) aus Beispiel 5 | 100 | 87 | 82 (*S*) | 91 (*S*) |
| 11 | R = 2,6-Dimethylphenyl *(S)* aus Beispiel 6 | 58 | 41 | 59 (*S*) | 74 (*S*) |

### Beispiel 12-13

### Hydrierung von Dimethylitakonat

In einem ausgeheizten Glasautoklaven werden 0,02 mmol Bis-Bicyclo[2.1.1]hepta-2,5-dien-rhodium(I)-terafluoroborat [Rh(nbd)₂]BF₄ und 0.04 mmol des entsprechenden Phosphits in 50 ml entgastem Methylenchlorid gelöst. Man lässt ca. 5 min rühren und gibt anschließend 200 mmol Dimethylitakonat und 5,000 g Diglyme in 250 ml entgastem Methylenchlorid zu. Nach dem Einstellen der jeweiligen Temperatur wird mit 0,5 bar Wasserstoffpartialdruck 2,5 h hydriert. Umsatz und ee werden gaschromatographisch bestimmt.

Die Ergebnisse sind in Tabelle 2 zusammengefasst.

**Tabelle 2**

| Beispiele | Ligand (Konfiguration) | Umsatz [%] | ee [%] Konfiguration |
|---|---|---|---|
| 12 | R = i-Propyl (S) aus Beispiel 2 | 90 | 97 (S) |
| 13 | R = (R)-1-Phenylethyl (S) aus Beispiel 4 | 100 | 99 (*S*) |

### Beispiel 14-18

### Hydrierung von 2-Acetamidoacrylsäuremethylester

0.0024 mmol des entsprechenden Phosphits werden eingewogen und unter Argon mit einer Lösung aus 0.0012 mmol Bis-Norbornadien-Rhodium(I)-hexafluorophosphat [(nbd)₂Rh]PF₆ und 0,12 mmol 2-Acetamidoacrylsäuremethylester in 0,8 ml entgastem CH₂Cl₂ versetzt. Anschließend wird bei 3 bar Wasserstoffdruck 23 h hydriert.

Die Ergebnisse sind in Tabelle 3 zusammengefasst.

**Tabelle 3**

| Beispiele | Ligand (Konfiguration) | Umsatz [%] | ee [%] Konfiguration |
|---|---|---|---|
| 14 | R = i-Propyl (*S*) aus Beispiel 2 | 100 | 94 (*R*) |
| 15 | R = Cyclohexyl (S) aus Beispiel 3 | 100 | 94 (*R*) |
| 16 | R = (*R*)-1-Phenylethyl (S) aus Beispiel 4 | 100 | 83 *(R)* |
| 17 | R = Phenyl (*S*) aus Beispiel 5 | 100 | 77 (*R*) |
| 18 | R = 2,6-Dimethylphenyl (S) aus Beispiel 6 | 100 | 18 (*R*) |

### Beispiel 19-20

### Hydrierung von (Z)-3-Acetamidobutensäuremethylester

0,0024 mmol des entsprechenden Phosphits werden eingewogen und unter Argon mit einer Lösung aus 0.0012 mmol Bis-Bicyclo[2.1.1]hepta-2,5-dien-rhodium(I)-hexafluorophosphat [Rh(nbd)₂Rh]BF₄ und 0,12 mmol (Z)-3-Acetamidobutensäuremethylester in 0,8 ml entgastem CH₂Cl₂ versetzt. Anschließend wird bei 3 bar Wasserstoffdruck 23 h hydriert.

Die Ergebnisse sind in Tabelle 4 zusammengefasst.

**Tabelle 4**

| Beispiele | Ligand (Konfiguration) | Umsatz [%] | ee [%] |
|---|---|---|---|
| 19 | R = i-Propyl (*S*) (Bsp. 2) | 4 | 71 |
| 20 | R = Cyclohexyl (S) (Bsp. 3) | 3 | 56 (andere Enantiomer |

### Beispiel 21-29

### Synthese von 3,3'-bis(1,1-dimethylethyl)-5,5',6,6'-tetramethyl-1,1'-Biphenyl-2,2'diol-Phosphite BIPHEN-Phosphite

In einem dreifach ausgeheizten und mit Argon gespülten 250 ml Schlenkkolben wurden 70 ml Toluol und 1,9 ml (0,0137 mol) Triethylamin auf -78°C gekühlt (Trockeneis/Aceton). 0,3 ml (0,0034 mol) Phosphortrichlorid wurde unter starkem Rühren zugesetzt. Zu dieser leicht trüben Suspension wurde im Argongegenstrom über einen Zeitraum von 2-3 Stunden 1 g festes (0,0028 mol) (R)- oder (S)-BIPHEN mittels eines Schütters hinzugegeben. Es bildete sich eine weiße oder gelbe Suspension, die über Nacht auf Raumtemperatur erwärmt wurde. Anschließend wurde der Ansatz unter Schutzgas über eine Umkehrfritte filtriert und das Lösungsmittel im Vakuum entfernt. Man erhielt einen gelben oder weißen Feststoff.

Eine Lösung von ca. 3 mmol (läq, 1,1g) dieses Feststoffes wurde in 41 ml Toluol, 0,43 ml (3 mmol, 1äq) Triethylamin und der in der Tabelle angeführten Menge Alkohol (Beispiele 21 bis 29) hinzugegeben. Die Lösung wurde über Nacht bei Raumtemperatur unter Argon gerührt. Anschließend wurde der Ansatz über eine Umkehrfritte vom Ammoniumsalz abfiltriert und das Lösungsmittel im Vakuum entfernt. Man erhielt einen gelben oder weißen Feststoff.

Die Ausbeuten und physikalischen Daten sind in Tabelle 5 zusammengefasst.

**Tabelle 5**

| Beispiel: | Ansatz | Ausbeute | Analytik |
|---|---|---|---|
| 21 | 0,26 g (0,0028 mol) | 0,911 g (68 %) gelber | ³¹P NMR: δ = 135,29 ppm |
| | Phenol | Feststoff | |
| 22 | 0,15 ml (0,0028 mol;1 | 0,802 g (67 %) weißer | ³¹P NMR: δ = 132,45 ppm |
| | äq) Ethanol | Feststoff | |
| 23¹⁾ | 0,34 g (0,0028 mol;1 | 1,353 g (95 %) weißer | ³¹P NMR: δ = 135,77 ppm |
| | äq) 2,6-Dimethylphenol | Feststoff | |
| | 0,14 g (0,0058 mol;1,1 | | |
| | äq) Natriumhydrid | | |
| 24 | 0,11 ml (0,0028 mol;1 | 1,151 g(99 %) weißer | ³¹P NMR δ = 130,18 ppm |
| | äq) Methanol | Feststoff | |
| 25 | 0,424 ml (0,0028 mol;1 | 0,978 g(65 %) gelber | ³¹P NMR δ = 136,3 ppm |
| | äq) tert-Buthylphenol | Feststoff | |
| 26 | 0,34 ml (0,0028 mol;1 | 0,881 g(62 %) weiß- | ³¹P NMR δ = 141,82 ppm |
| | äq) Phenethyalkohol | gelber Feststoff | und 138,58 (Diastereomerenpaar). |
| 27 | 0,52 g (0,0028 mol;1 | 0,771 g (48 %) weißer | ³¹P NMR δ = 136,35 ppm |
| | äq) Diphenylcarbinol | Feststoff | |
| 28 | 0,22 ml (0,0028 mol;1 | 0,521 g (33 %) weißen | ³¹P NMR: δ 142,84 ppm |
| | äq) Isopropanol | Feststoff | |
| 29¹⁾ | Fünffacher Ansatz 8,1 | 7,23 g (92 %) gelber | ³¹P NMR: δ 1365,74 ppm |
| | ml (0,014mol; äq) 2,6- | Feststoff | |
| | Diisopropylphenol | | |

| | | | |
|---|---|---|---|
| ¹⁾ Zusätzlicher Einsatz von Natriumhydrid als Base zur Bildung des Phenolats | | | |

### Beispiele 30 bis 33

### Asymmetrische Hydrierung mit BIPHEN Phosphiten

Die Liganden (aus den Beispielen 21 und 24) wurden in die Reaktionsgefäße eingewogen (Ansatzgröße: 0.09 mmol). Die Substrate wurden anschließend als Stammlösung (Verdünnung 0,13 mol/l) in je 5,1 ml Methylenchlorid vorbereitet und entgast. 5,6 mg Bis-(1,5-Cycloctadien)Rhodium-Triflat Rh(COD)₂OTf wurde jeweils zugegeben und erneut entgast. In der Glovebox wurden je 0,72 ml Lösung auf die einzelnen Gefäße verteilt (2 mol % Katalysator und 2 mol % Ligand). Alle Ansätze wurden in einem Autoklaven hydriert (23h, 3bar Wasserstoffdruck RT).

Die Ergebnisse sind in den Tabellen 6 und 7 zusammengefasst.

**Tabelle 6**

| **Hydrierung von (Z)-3-Acetamidobutensäuremethylester** | | |
|---|---|---|
| Ligand | 21 | 24 |
| Umsatz [%] | 100 | 100 |
| ee [%] | 11.3 | 59 |
| Beispiel Nr. | 30 | 31 |

**Tabelle 7**

| **Hydrierung von Dimethylitakonat** | | |
|---|---|---|
| Ligand | 21 | 24 |
| Umsatz [%] | 100 | 100 |
| ee [%] | 14 | 13 |
| Beispiel Nr. | 32 | 33 |

## Patentansprüche

1. Chirale Monophosphorverbindungen der allgemeinen Formel (I), 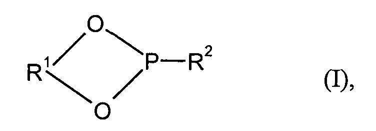 in der
R¹ für einen unsubstituierten oder substituierten 1,1'-Biphenyl-2,2'-diyl-Rest steht und
R² für einen Rest steht, der ausgewählt ist aus der Gruppe substituiertes oder unsubstituiertes Alkyl, substituiertes oder unsubstituiertes Aryl, substituiertes oder unsubstituiertes Alkoxy, substituiertes oder unsubstituiertes Aryloxy oder tertiäres Amino.

2. Chirale Monophosphorverbindungen gemäß Anspruch 1, **dadurch gekennzeichnet, dass** in der allgemeinen Formel (I)
R¹ für einen unsubstituierten oder substituierten 1,1'-Biphenyl-2,2'-diyl-Rest steht und
R² für einen Rest steht, der ausgewählt ist aus der Gruppe substituiertes oder unsubstituiertes Alkoxy, substituiertes oder unsubstituiertes Aryloxy oder tertiäres Amino.

3. Chirale Monophosphorverbindungen gemäß Anspruch 1, **dadurch gekennzeichnet, dass** in der allgemeinen Formel (I)
R¹ für einen unsubstituierten oder substituierten 1,1'-Biphenyl-2,2'-diyl-Rest der allgemeinen Formel (VI) steht, 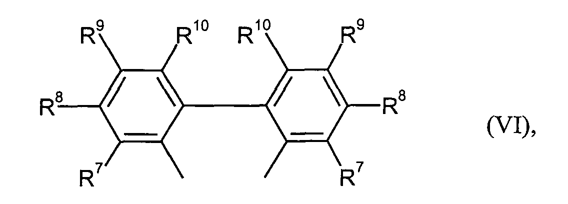
in der die Reste R⁷, R⁸, R⁹ und R¹⁰ jeweils unabhängig voneinander ausgewählt sind aus der Gruppe Fluor, Chlor, Brom, unsubstituiertes oder substituiertes geschütztes Hydroxy, unsubstituiertes oder substituiertes C₁-C₆-Alkyl, unsubstituiertes oder substituiertes C₁-C₆-Alkoxy, unsubstituiertes oder substituiertes C₁-C₆-Alkylthio, Cyano, freies oder geschütztes Formyl, unsubstituiertes oder substituiertes C₆-C₁₂-Aryl, Tri(C₁-C₆-alkyl)siloxyl oder Resten der allgemeinen Formel (II)
A-B-D-E (II),
in der unabhängig voneinander
A fehlt oder für einen C₁-C₈-Alkylenrest steht und
B fehlt oder für Sauerstoff, Schwefel oder NR³ steht,
wobei
R³ Wasserstoff, C₁-C₁₆-Alkyl oder C₆-C₁₀-Aryl bedeutet und
D für eine Carbonyl-Gruppe steht und
E für R⁴, OR⁴, NHR⁵ oder NR⁵R⁶ steht,
wobei
R⁴ für C₁-C₁₂-Alkyl oder C₆-C₁₀-Aryl und
R₅ und R₆ jeweils unabhängig voneinander für C₁-C₈-Alkyl oder C₆-C₁₀-Aryl stehen oder NR⁵R⁶ zusammen für einen cyclischen Aminorest steht,
oder Resten der allgemeinen Formeln (IIIa)und (IIIb) steht
A-E (IIIa)
A-COX (IIIb),
in denen A und E die oben angegebene Bedeutung besitzen und X für OH, NH₂ oder OM steht, wobei M ein Alkalimetallion, ein halbes Äquivalent eines Erdalkalimetallions, ein Ammoniumion oder ein organisches Ammoniumion bedeutet
oder
die beiden Reste R¹⁰ zusammen verbrückend sind.

4. Chirale Monophosphorverbindungen gemäß Anspruch 1, **dadurch gekennzeichnet, dass** in der allgemeinen Formel (I)
R¹ für einen der folgenden Reste steht: 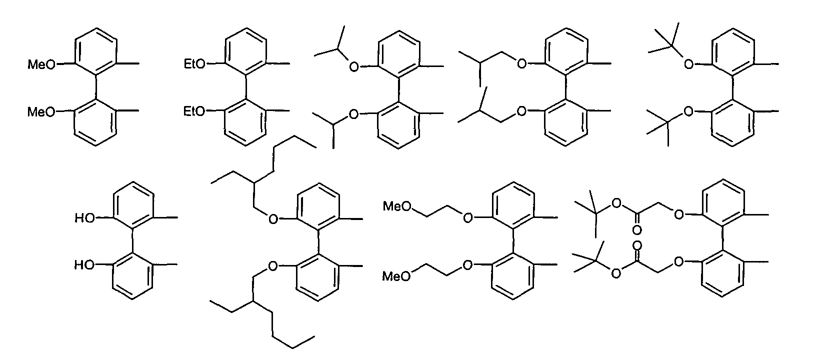 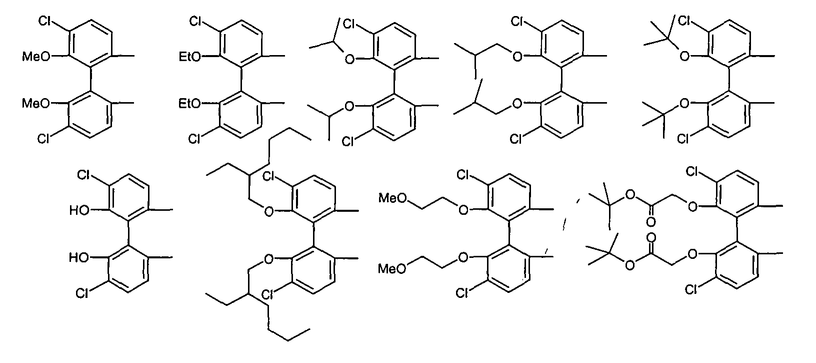
oder R¹ für solche Reste der allgemeinen Formel (VIII) 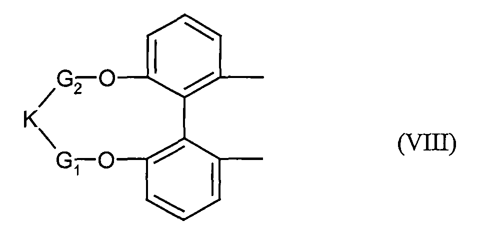 in der G¹-K-G² zusammen für
(R)-1,2-Propandiyl, (S,S)-1,2-Cyclohexandiyl, (R,R)-1,2-Cyclohexandiyl, 1,2-Ethandiyl, 1,3-Propandiyl, 1,4-Butandiyl oder 1,4-Dioxobutandiyl steht
oder R¹ für folgende Reste steht:

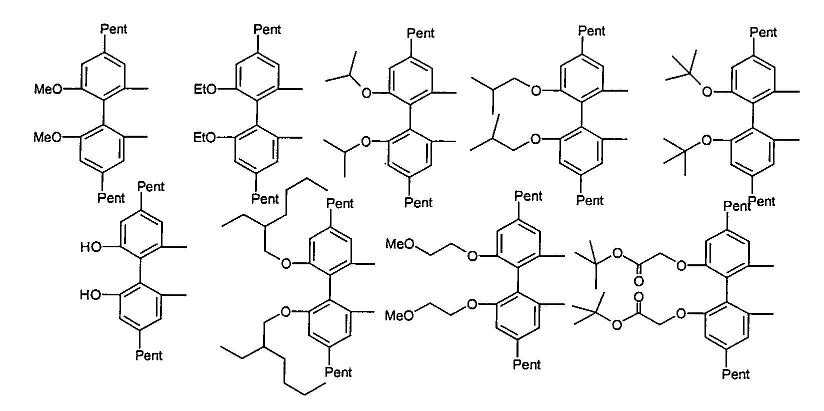

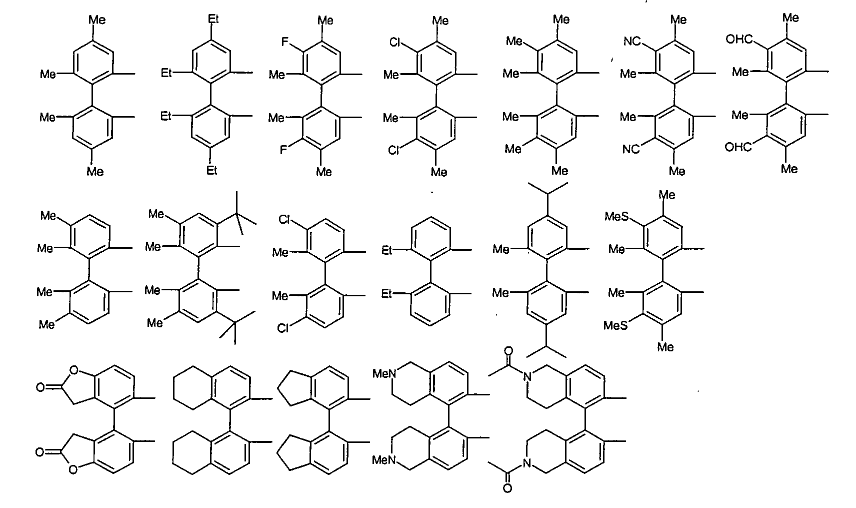

5. Chirale Monophosphorverbindungen gemäß Anspruch 1, **dadurch gekennzeichnet, dass** in der allgemeinen Formel (I)
R² für Methoxy, Ethoxy, iso-Propoxy, Cyclohexyloxy, Phenoxy, (R)-1-Phenylethoxy oder (S)-1-Phenylethoxy steht.

6. ((S)-5,5'-Dichlor-6,6'-dimethoxy-1,1'-biphenyl-2,2'-diyl)-isopropyl-phosphit
((R)-5,5'-Dichlor-6,6'-dimethoxy-1,1'-biphenyl-2,2'-diyl)-isopropyl-phosphit
((R)-5,5'-Dichlor-6,6'-dimethoxy-1,1'-biphenyl-2,2'-diyl)-(R)-1-phenylethylphosphit
((R)-5,5'-Dichlor-6,6'-dimethoxy-1,1'-biphenyl-2,2'-diyl)-(S)-1-phenylethylphosphit
((S)-5,5 '-Dichlor-6,6'-dimethoxy-1,1'-biphenyl-2,2'-diyl)-(R)-1-phenylethylphosphit
((S)-5,5 '-Dichlor-6,6'-dimethoxy-1,1'-biphenyl-2,2'-diyl)-(S)-1-phenylethylphosphit
((S)-5,5'-Dichlor-6,6'-dimethoxy-1,1'-biphenyl-2,2'-diyl)-cyclohexyl-phosphit
((R)-5,5 '-Dichlor-6,6'-dimethoxy-1,1'-biphenyl-2,2'-diyl)-cyclohexyl-phosphit
((S)-5,5'-Dichlor-6,6'-dimethoxy-1,1'-biphenyl-2,2'-diyl)-phenyl-phosphit
((R)-5,5'-Dichlor-6,6'-dimethoxy-1,1'-biphenyl-2,2'-diyl)-phenyl-phosphit
((S)-5,5'-Dichlor-6,6'-dimethoxy-1,1'-biphenyl-2,2'-diyl)-2,6-dimethylphenyl-phosphit
((R)-5,5 '-Dichlor-6,6'-dimethoxy-1,1'-biphenyl-2,2'-diyl)-2,6-dimethylphenyl-phosphit
((S)-5,5',6,6'-tetramethyl-3,3 '-bis(tert.-butyl)-1,1'-biphenyl-2,2'-diyl)-isopropyl-phosphit
((R)-5,5',6,6'-tetramethyl-3,3'-bis(tert.-butyl)-1,1'-biphenyl-2,2'-diyl)-isopropyl-phosphit
((S)-5,5',6,6'-tetramethyl-3,3'-bis(tert.-butyl)-1,1 '-biphenyl-2,2'-diyl)-(rac)-1 -phenylethyl-phosphit
((R)-5,5',6,6'-tetramethyl-3,3'-bis(tert.-butyl)-1,1'-biphenyl-2,2'-diyl)-(rac)-1 -phenylethyl-phosphit
((S)-5,5',6,6'-tetramethyl-3,3'-bis(tert.-butyl)-1,1'-biphenyl-2,2'-diyl)-(S)-1-phenylethyl-phosphit
((R)-5,5',6,6'-tetramethyl-3,3 '-bis(tert.-butyl)-1,1'-biphenyl-2,2'-diyl)-(S)-1-phenylethyl-phosphit
((S)-5,5',6,6'-tetramethyl-3,3'-bis(tert.-butyl)-1,1'-biphenyl-2,2'-diyl)-(R)-1-phenylethyl-phosphit
((R)-5,5',6,6'-tetramethyl-3,3'-bis(tert.-butyl)-1,1'-biphenyl-2,2'-diyl)-(R)-1-phenylethyl-phosphit
((S)-5,5',6,6'-tetramethyl-3,3'-bis(tert.-butyl)-1,1'-biphenyl-2,2'-diyl)-diphenylmethyl-phosphit
((R)-5,5',6,6'-tetramethyl-3,3'-bis(tert.-butyl)-1,1'-biphenyl-2,2'-diyl)-diphenylmethyl-phosphit
((S)-5,5',6,6'-tetramethyl-3,3'-bis(tert.-butyl)-1,1'-biphenyl-2,2'-diyl)-methyl-phosphit
((R)-5,5',6,6'-tetramethyl-3,3'-bis(tert.-butyl)-1,1'-biphenyl-2,2'-diyl)-methyl-phosphit
((S)-5,5',6,6'-tetramethyl-3,3'-bis(tert.-butyl)-1,1'-biphenyl-2,2'-diyl)-2,6-dimethylphenyl-phosphit
((R)-5,5',6,6'-tetramethyl-3,3'-bis(tert.-butyl)-1,1'-biphenyl-2,2'-diyl)-2,6-dimethylphenyl-phosphit
((S)-5,5',6,6'-tetramethyl-3,3'-bis(tert.-butyl)-1,1'-biphenyl-2,2'-diyl)-2,6-diiso-propylphenyl-phosphit
((R)-5,5',6,6'-tetramethyl-3,3'-bis(tert.-butyl)-1,1'-biphenyl-2,2'-diyl)-2,6-diiso-propylphenyl-phosphit
((S)-5,5',6,6'-tetramethyl-3,3'-bis(tert.-butyl)-1,1'-biphenyl-2,2'-diyl)-phenyl-phosphit
((R)-5,5',6,6'-tetramethyl-3,3'-bis(tert.-butyl)-1,1'-biphenyl-2,2'-diyl)-phenyl-phosphit
((S)-5,5',6,6'-tetramethyl-3,3'-bis(tert.-butyl)-1,1'-biphenyl-2,2'-diyl)-ethyl-phosphit
((R)-5,5',6,6'-tetramethyl-3,3'-bis(tert.-butyl)-1,1'-biphenyl-2,2'-diyl)-ethyl-phosphit
((S)-5,5',6,6'-tetramethyl-3,3'-bis(tert.-butyl)-1,1'-biphenyl-2,2'-diyl)-4-tert-butylphenyl-phosphit
((R)-5,5',6,6'-tetramethyl-3,3'-bis(tert.-butyl)-1,1'-biphenyl-2,2'-diyl)-4-tert-butylphenyl-phosphit

7. Verbindungen der allgemeinen Formel (XI) 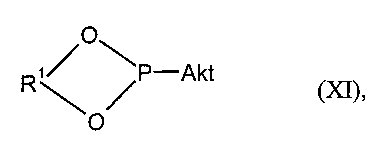 in der R¹ für einen unsubstituierten oder substituierten 1,1'-Biphenyl-2,2'-diyl-Rest steht und
Akt für Chlor, Brom, Iod oder Dialkylamino steht.

8. Verwendung von Verbindungen gemäß Anspruch 7 zur Herstellung von chiralen Phosphoniten, chiralen Phosphoramiditen und chiralen Phosphiten.

9. Verwendung von chiralen Monophosphorverbindungen gemäß einem der Ansprüche 1 bis 7 in der asymmetrischen Synthese.

10. Verwendung von chiralen Monophosphorverbindungen gemäß einem der Ansprüche 1 bis 7 für asymmetrische 1,4-Additionen, asymmetrische Hydroformylierungen, asymmetrische Hydrocyanierungen, asymmetrische Heck-Reaktionen und asymmetrische Hydrogenierungen.

11. Verwendung von chiralen Monophosphorverbindungen gemäß einem der Ansprüche 1 bis 7 für asymmetrische Hydrogenierungen.

12. Verwendung von chiralen Monophosphorverbindungen gemäß einem der Ansprüche 1 bis 7 für asymmetrische Hydrogenierungen von prochiralen Olefinen oder prochiralen Enaminen.

13. Katalysatoren, enthaltend Übergangsmetallkomplexe von chiralen Monophosphorverbindungen der allgemeinen Formel (I) 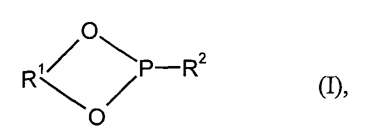 in der
R¹ für einen unsubstituierten oder substituierten 1,1'-Biphenyl-2,2'-diyl-Rest steht und
R² für einen Rest steht, der ausgewählt ist aus der Gruppe substituiertes oder unsubstituiertes Alkyl, substituiertes oder unsubstituiertes Aryl, substituiertes oder unsubstituiertes Alkoxy, substituiertes oder unsubstituiertes Aryloxy oder tertiäres Amino.

14. Katalysatoren gemäß Anspruch 13, **dadurch gekennzeichnet, dass** sie aus chiralen Monophosphorverbindungen der allgemeinen Formel (I) und Metallverbindungen erzeugt werden.

15. Verfahren zur Herstellung chiraler Verbindungen, **dadurch gekennzeichnet, dass** als Katalysatoren solche gemäß den Ansprüchen 13 bis 14 eingesetzt werden.

16. Verfahren zur asymmetrischen Hydrierung von Substraten, **dadurch gekennzeichnet, dass** als Katalysatoren solche gemäß den Ansprüchen 13 bis 14 eingesetzt werden.

17. Verfahren gemäß Anspruch 16, **dadurch gekennzeichnet, dass** als Katalysatoren solche eingesetzt werden, die aus chiralen Monophosphorverbindungen gemäß den Ansprüchen 1 bis 7 und Metallverbindungen der allgemeinen Formel (XIIIe)
[M(B³)₂]An (XIIIe),
in der
M für Rhodium und
B³ für ein (C₄-C₁₂)-Dien wie beispielsweise Norbornadien oder 1,5-Cyclooctadien stehen und
An für ein nicht oder schwach koordinierendes Anion steht,
erzeugt werden.

18. Verfahren nach Ansprüchen 16 bis 17, **dadurch gekennzeichnet, dass** es in mindestens einem Lösungsmittel ausgewählt aus der Gruppe chlorierte Alkane, C₁-C₆-Alkohole aromatische Kohlenwasserstoffe, Ketone und Carbonsäureester durchgeführt wird.

19. Verfahren nach Ansprüchen 16 bis 18, **dadurch gekennzeichnet, dass** es bei Temperaturen von -20 bis 200°C durchgeführt wird.

20. Verfahren nach Ansprüchen 16 bis 19, **dadurch gekennzeichnet, dass** es bei einem Wasserstoffdruck von 0,1 bis 200 bar durchgeführt wird.

21. Verfahren nach Ansprüchen 16 bis 20, **dadurch gekennzeichnet, dass** die Menge der eingesetzten Metallverbindung oder des eingesetzten Übergangsmetallkomplexes 0,001 bis 5 mol-% bezogen auf das eingesetzte Substrat beträgt.

22. Verfahren nach einem oder mehreren der Ansprüche 15 bis 21, **dadurch gekennzeichnet, dass** die Substrat-Umsatzrate über 1000 h⁻¹ beträgt.

23. Verwendung von Verbindungen, die nach einem Verfahren gemäß einem oder mehreren der Ansprüche 15 bis 20 hergestellt wurden, zur Herstellung von chiralen Wirkstoffen in Arzneimitteln und Agrarchemikalien, oder Zwischenprodukten dieser beiden Klassen.
